# EUROPEAN PATENT APPLICATION

(11) **EP 3 231 358 A1**
(43) Date of publication of application: **18.10.2017**
(21) Application number: 17166337.0
(22) Date of filing: 12.04.2017
(51) Int. Cl.: A61B 5/00, A61B 5/042

(54) **BASKET CATHETER WITH PRESTRAINED FRAMEWORK**

(30) Priority: 13.04.2016 US 201615098175
(71) Applicant: Biosense Webster (Israel), Ltd., Yokneam, 2066717 (IL)
(72) Inventor: WU, Steven, Irwindale, CA 91706 (US); MIN, Sungwoo, Irwindale, CA 91706 (US); AUJLA, Vishav Manak Singh, Irwindale, CA 91706 (US); MERCHANT, Neil, Irwindale, CA 91706 (US)
(74) Representative: Carpmaels & Ransford LLP

(57) **Abstract**

A catheter having a basket-shaped electrode assembly at the distal end of the catheter body formed from a plurality of spines with electrodes. The plurality of spines are formed by a framework which is prestrained to have a diameter greater than the diameter of the expanded arrangement of the basket-shaped electrode assembly and a length less than the length of the expanded arrangement of the basket-shaped electrode assembly.

## Description

### FIELD OF THE PRESENT DISCLOSURE

This invention relates to electrophysiologic (EP) catheters for mapping and/or ablation in the heart.

### BACKGROUND

Mapping of electrical potentials in the heart is now commonly performed, using cardiac catheters comprising electrophysiological sensors for mapping the electrical activity of the heart. Typically, time-varying electrical potentials in the endocardium are sensed and recorded as a function of position inside the heart, and then used to map a local electrogram or local activation time. Activation time differs from point to point in the endocardium due to the time required for conduction of electrical impulses through the heart muscle. The direction of this electrical conduction at any point in the heart is conventionally represented by an activation vector, which is normal to an isoelectric activation front, both of which may be derived from a map of activation time. The rate of propagation of the activation front through any point in the endocardium may be represented as a velocity vector. Mapping the activation front and conduction fields aids the physician in identifying and diagnosing abnormalities, such as ventricular and atrial tachycardia and ventricular and atrial fibrillation, which may result from areas of impaired electrical propagation in the heart tissue.

Localized defects in the heart's conduction of activation signals may be identified by observing phenomena such as multiple activation fronts, abnormal concentrations of activation vectors, or changes in the velocity vector or deviation of the vector from normal values. Examples of such defects include re-entrant areas, which may be associated with signal patterns known as complex fractionated electrograms. Once a defect is located by such mapping, it may be ablated (if it is functioning abnormally) or otherwise treated so as to restore the normal function of the heart insofar as is possible. As an illustration, cardiac arrhythmias including atrial fibrillation, may occur when regions of cardiac tissue abnormally conduct electric signals to adjacent tissue, thereby disrupting the normal cardiac cycle and causing asynchronous rhythm. Procedures for treating arrhythmia include disrupting the origin of the signals causing the arrhythmia, as well as disrupting the conducting pathway for such signals, such as by forming lesions to isolate the aberrant portion. Thus, by selectively ablating cardiac tissue by application of energy via a catheter, it is sometimes possible to cease or modify the propagation of unwanted electrical signals from one portion of the heart to another. The ablation process destroys the unwanted electrical pathways by formation of non-conducting lesions.

A number of advantages may be obtained by providing a catheter having multiple electrodes to allow for mapping larger regions and/or for creating a plurality of lesions either simultaneously or without the need to reposition the catheter. One suitable configuration is a basket-shaped electrode assembly, such as described in commonly assigned U.S. Pat. Nos. 5,772,590, 6,748,255 and 6,973,340, the entire disclosures of each of which are incorporated herein by reference. Basket catheters typically have an elongated catheter body and a basket-shaped electrode assembly mounted at the distal end of the catheter body. The basket assembly has proximal and distal ends and comprises a plurality of spines connected at their proximal and distal ends. Each spine comprises at least one electrode. The basket assembly has an expanded arrangement wherein the spines bow radially outwardly and a collapsed arrangement wherein the spines are arranged generally along the axis of the catheter body.

For diagnostic purposes, it is desirable that a basket-shaped electrode assembly be capable of detecting in as few beats as possible, including a single beat, as much of the electrical function of the region in which the electrode assembly is deployed, such as the left or right atrium. To reliably achieve this goal, the basket should deploy into a specific configuration that positions the spines to obtain uniform coverage of the tissue in the region of interest with the electrodes carried by the spines. Similarly, when employing a basket catheter to deliver energy for a therapeutic procedure, such as tissue ablation, achieving a specific spine configuration when the basket assembly is deployed helps ensure that one or more of the electrodes carried by the spines are positioned at the intended treatment site. For many basket catheters, it is desirable for the basket-shaped electrode assembly to have a spherical configuration when assuming the expanded arrangement to achieve these goals.

The configuration of the basket-shaped electrode assembly when expanded may be imparted by an internal framework of resilient material that assumes the expanded arrangement when unconstrained, such as when the basket-shaped electrode assembly is advanced out of a guide catheter. However, conventional catheter designs may have difficulty achieving the desired spherical configuration due to the resistance created by the other components of the spine that cover the framework. For example, a nonconductive tubing may be disposed over the spines of the framework upon which the electrodes are deployed. Further, depending on the number of electrodes carried by each spine, a significant amount of cabling may be necessary to provide the requisite electrical connections. These and other may create the noted resistance, which tends to maintain the spines in a straightened configuration rather than bowing radially outwards into the desired spherical configuration. Correspondingly, even though conventional basket catheters may be intended to assume a spherical configuration when deployed into an expanded arrangement, the resistance of the spines may cause the basket-shaped electrode assembly to assume or more elliptical configuration than spherical, such that the longitudinal axis of the basket-shaped electrode assembly (i.e., the distance from proximal pole to distal pole) is greater than the diameter. As a result, such conventional basket-shaped electrode assemblies may not be spherical when deployed and may not achieve the desired coverage.

Accordingly, it would be desirable to provide a framework for a basket-shaped electrode assembly that ameliorates these issues. Notably, it would be desirable to provide a framework that compensates for the resistance imparted by spine components. Similarly, it would be desirable to provide a framework that assumes the intended configuration when the basket-shaped electrode assembly is deployed in its expanded arrangement. The techniques of this disclosure as described in the following materials satisfy these and other needs.

### SUMMARY

The present disclosure is directed to a catheter having an elongated catheter body with proximal and distal ends and a basket-shaped electrode assembly at the distal end of the catheter body. The basket-shaped electrode assembly may include a plurality of spines connected at their proximal and distal ends, each spine comprising a plurality of electrodes. The basket-shaped electrode assembly may have an expanded arrangement having a length and a diameter in which the spines bow radially outward and a collapsed arrangement in which the spines are arranged generally along a longitudinal axis of the catheter body. The spines may be formed by a framework which is prestrained to have a diameter greater than the diameter of the expanded arrangement of the basket-shaped electrode assembly and a length less than the length of the expanded arrangement of the basket-shaped electrode assembly.

In one aspect, the framework may be formed from a shape memory material. The framework may be monolithic and formed from a cut tube of material.

In one aspect, the spines may include a nonconductive covering. The length of the expanded arrangement may be greater than the length of the prestrained framework and the diameter of the expanded arrangement may be less than the diameter of the prestrained framework at least in part due to the nonconductive covering.

In one aspect, the expanded arrangement of the basket-shaped electrode assembly may have an approximately spherical configuration. Accordingly, the diameter of the prestrained framework may be greater than the length of the prestrained framework. In another embodiment, the expanded arrangement of the basket-shaped electrode assembly may have an approximately elliptical configuration, such that. Accordingly, the diameter of the prestrained framework may be equal or less than the length of the prestrained framework. Depending on the embodiment, the ratio of the diameter of the prestrained framework to the length of the prestrained framework may be in the range of approximately 2:1 to 8:10.

This disclosure also includes a framework for a basket-shaped electrode assembly. The framework may have plurality of flexible cores for spines of the basket-shaped electrode assembly, wherein the framework has a prestrained diameter greater than a diameter of an expanded arrangement of the basket-shaped electrode assembly and a prestrained length less than a length of an expanded arrangement of the basket-shaped electrode assembly. The prestrained diameter of the framework may be less than the prestrained length of the framework. Depending on the embodiment, the ratio of the diameter of the prestrained framework to the length of the prestrained framework may be in the range of approximately 2:1 to 8:10.

This disclosure also includes a method for treatment that may involve providing a catheter having an elongated catheter body with proximal and distal ends and a basket-shaped electrode assembly at the distal end of the catheter body, the basket-shaped electrode assembly comprising a plurality of spines connected at their proximal and distal ends, each spine comprising a plurality of electrodes, wherein the spines are formed by a framework which is prestrained to have a diameter and a length, advancing the distal end of the catheter with the basket-shaped electrode assembly to a desired region within a patient with the interconnected framework in a collapsed arrangement in which the spines are arranged generally along a longitudinal axis of the catheter body and causing the basket-shaped electrode assembly to assume an expanded arrangement in which the spines bow radially outwards from the longitudinal axis of the catheter body so that at least one electrode is in contact with tissue, wherein the expanded arrangement has a length greater than the length of the prestrained framework and the expanded arrangement has a diameter less than the diameter of the prestrained framework.

In one aspect, electrical signals may be received from the at least one electrode in contact with tissue. Alternatively or in addition, radio frequency energy may be delivered to the at least one electrode in contact with tissue to form a lesion.

This disclosure also includes a method for manufacturing a basket-shaped electrode with a length and a diameter when in an expanded arrangement and having a plurality of spines connected at their proximal and distal ends. The method may involve prestraining a framework to have a diameter greater than the diameter of the expanded arrangement of the basket-shaped electrode assembly and a length less than the length of the expanded arrangement of the basket-shaped electrode assembly, wherein the framework forms the spines of the basket-shaped electrode assembly.

In one aspect, components may be applied to the spines which cause the length of the expanded arrangement to be greater than the length of the prestrained framework and cause the diameter of the expanded arrangement to be less than the diameter of the prestrained framework. The components may include a nonconductive covering for the spines.

In one aspect, prestraining the framework may involve prestraining the framework to have the diameter of the framework greater than the length of the framework so that the basket-shaped electrode assembly has an approximately spherical configuration when assuming the expanded arrangement.

### BRIEF DESCRIPTION OF THE DRAWINGS

Further features and advantages will become apparent from the following and more particular description of the preferred embodiments of the disclosure, as illustrated in the accompanying drawings, and in which like referenced characters generally refer to the same parts or elements throughout the views, and in which:
FIG. 1 is a top plan view of a basket-shaped electrode assembly catheter of the present invention, according to one embodiment.
FIG. 2 is a detail of a spine of a basket-shaped electrode assembly, according to one embodiment.
FIG. 3 is a schematic view of a prestrained framework for a spherical basket-shaped electrode assembly, according to one embodiment.
FIG. 4 is a schematic view of a prestrained framework for an elliptical basket-shaped electrode assembly, according to one embodiment.
FIG. 5 is a schematic view of a basket-shaped electrode assembly having a prestrained framework within the left atrium, according to one embodiment.

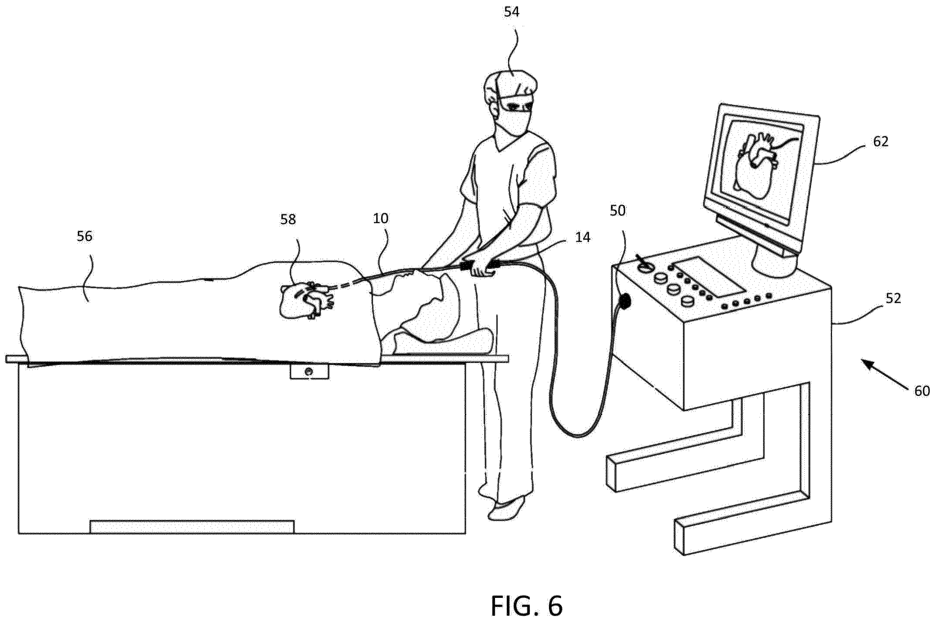

FIG. 6 is a schematic illustration of an invasive medical procedure using a basket-shaped electrode assembly having a prestrained framework, according to one embodiment.

### DETAILED DESCRIPTION

At the outset, it is to be understood that this disclosure is not limited to particularly exemplified materials, architectures, routines, methods or structures as such may vary. Thus, although a number of such options, similar or equivalent to those described herein, can be used in the practice or embodiments of this disclosure, the preferred materials and methods are described herein.

It is also to be understood that the terminology used herein is for the purpose of describing particular embodiments of this disclosure only and is not intended to be limiting.

The detailed description set forth below in connection with the appended drawings is intended as a description of exemplary embodiments of the present disclosure and is not intended to represent the only exemplary embodiments in which the present disclosure can be practiced. The term "exemplary" used throughout this description means "serving as an example, instance, or illustration," and should not necessarily be construed as preferred or advantageous over other exemplary embodiments. The detailed description includes specific details for the purpose of providing a thorough understanding of the exemplary embodiments of the specification. It will be apparent to those skilled in the art that the exemplary embodiments of the specification may be practiced without these specific details. In some instances, well known structures and devices are shown in block diagram form in order to avoid obscuring the novelty of the exemplary embodiments presented herein.

For purposes of convenience and clarity only, directional terms, such as top, bottom, left, right, up, down, over, above, below, beneath, rear, back, and front, may be used with respect to the accompanying drawings. These and similar directional terms should not be construed to limit the scope of the disclosure in any manner.

Unless defined otherwise, all technical and scientific terms used herein have the same meaning as commonly understood by one having ordinary skill in the art to which the disclosure pertains.

Finally, as used in this specification and the appended claims, the singular forms "a, "an" and "the" include plural referents unless the content clearly dictates otherwise.

As noted above, certain types of electrical activity within a heart chamber are not cyclical. Examples include arterial flutter or arterial fibrillation, and ventricular tachycardia originating in scars in the wall of the ventricle that have resulted from infarcts. Such electrical activity is random from beat to beat. To analyze or 'map' this type of electrical activity, it is desirable to obtain the 'picture' as quickly as possible, such as within one heartbeat. In other words, all the points of the map or picture may be obtained simultaneously within one-tenth of a second. According to the techniques of this disclosure, an assembly having multiple spines may deploy an array of electrodes to accurately map this electrical activity.

Further, RF energy may be delivered to selected treatment areas for ablation based therapies, including for example, isolation of a source of irregular electrical signals by blocking electrical conduction. Focal ablations using unipolar devices benefit from targeted delivery of RF energy along with localized feedback of catheter placement, both spatially and with respect to tissue engagement. However, focal ablation procedures typically involve relative long procedure times as a result of the physician needing to stitch a series of "quantized" RF ablation to form a lesion having the desired characteristics, such as the creation of a continuous circumferential block which surrounds the ostium of the targeted vein. Additionally, the use of a focal unipolar electrode requires substantial physician skill level augmented with peripheral navigation systems in order to accurately and reliably position the electrodes. Correspondingly, an assembly having multiple spines may deploy an array of electrodes to simultaneously deliver ablation energy at a plurality of locations.

According to the techniques of this disclosure, a basket-shaped electrode assembly may assume a desired expanded arrangement, such as a spherical configuration, to conform more closely to the anatomy of the patient's heart in order to accurately map this electrical activity or to deliver energy to a treatment area being targeted. By employing a prestrained framework, resistance imparted by components of the spine may be overcome to allow the basket-shaped electrode assembly to deploy into the intended configuration to more closely conform to the patient's anatomy.

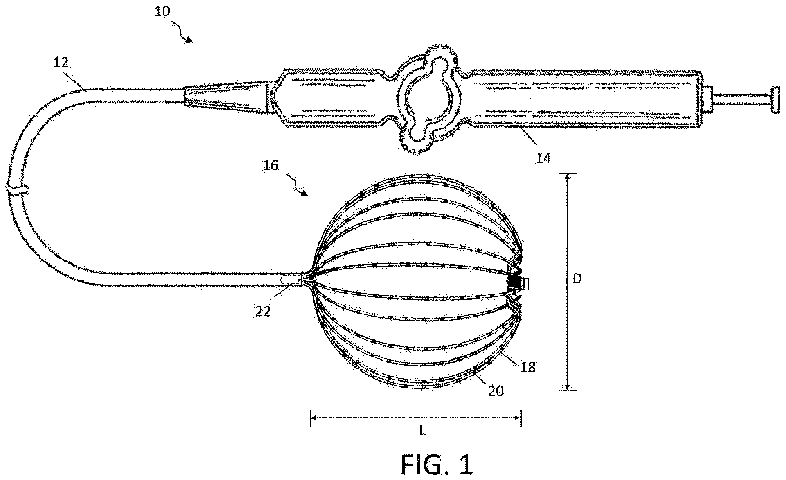

To help illustrate aspects of this disclosure, FIG. 1 depicts a catheter 10 with an elongated catheter body 12 having proximal and distal ends and a control handle 14 at the proximal end of the catheter body, with a basket-shaped electrode assembly 16 having a plurality of spines 18, each carrying multiple electrodes 20, mounted at the distal end of the catheter body 12. The catheter body 12 comprises an elongated tubular construction having a single, axial or central lumen (not shown), but can optionally have multiple lumens if desired. Any number of spines 18 may be employed. For example, a basket-shaped electrode assembly having a relatively high density electrode array may have eight, ten, twelve or more spines. Depending on the application, fewer spines, such as two or more may be used. Spines 18 may be evenly or unevenly distributed radially. Further, each spine 18 may include multiple electrodes 20, such in the range of ten to twenty electrodes per spine. In other applications, fewer numbers electrodes may be employed as desired. Further, the electrodes may be evenly distributed along each spine or may be skewed proximally, centrally or distally to facilitate analysis of the measured electrical signals or to access desired regions of the patient's anatomy. In some embodiments, one or more of electrodes 20 may be configured to deliver radio frequency energy to ablate tissue adjacent the electrode.

The catheter body 12 is flexible, i.e., bendable, but substantially non-compressible along its length. The catheter body 12 can be of any suitable construction and made of any suitable material. One construction comprises an outer wall made of polyurethane or PEBAX® (polyether block amide). The outer wall comprises an imbedded braided mesh of stainless steel or the like to increase torsional stiffness of the catheter body 12 so that, when the control handle 14 is rotated, the distal end of the catheter body will rotate in a corresponding manner. The outer diameter of the catheter body 12 is not critical, but generally should be as small as possible and may be no more than about 10 french depending on the desired application. Likewise, the thickness of the outer wall is not critical, but may be thin enough so that the central lumen can accommodate a pulling member wire, lead wires, sensor cables and any other wires, cables or tubes. If desired, the inner surface of the outer wall is lined with a stiffening tube (not shown) to provide improved torsional stability. An example of a catheter body construction suitable for use in connection with the present invention is described and depicted in U.S. Pat. No. 6,064,905, the entire disclosure of which is incorporated herein by reference. Additionally, one or more location sensors 22 may be provided near a distal end of the catheter 10 adjacent the basket-shaped electrode assembly 16 as schematically indicated in FIG. 1. The sensor(s) may each comprise a magnetic-field-responsive coil or a plurality of such coils. Using a plurality of coils enables six-dimensional position and orientation coordinates to be determined. The sensors may therefore generate electrical position signals in response to the magnetic fields from external coils to enable a position determination (e.g., the location and orientation) of the distal end of catheter 10 within the heart cavity to be made.

The basket-shaped electrode assembly 16 may have a preshaped expanded arrangement that is assumed when spines 18 are unconstrained. In the expanded arrangement, spines 18 bow radially outwards, so that basket-shaped electrode assembly has a longitudinal length, L, and a diameter, D. Spines 18 may also assume a collapsed arrangement, such as when constrained by a guiding sheath, having a generally linear alignment with the catheter body 12 to minimize the outer diameter for insertion within and withdrawal from the patient. As noted above, the expanded arrangement may bring electrodes 20 into contract or closer proximity with the walls of the chamber or other region in which basket-shaped electrode assembly 16 is positioned, for example by having a spherical configuration in which L is approximately equal to D. The overall size of basket-shaped electrode assembly 16 may be selected based on the patient's anatomy to provide a close fit to the area of the patient being investigated or treated, such as the right or left atria.

Basket-shaped electrode assembly 16 may be constructed by employing a framework of a suitable substrate material. In one aspect, a shape memory material may be used to aid assuming the expanded and collapsed arrangements. For example, nickel-titanium alloys known as nitinol may be used. At body temperature, nitinol wire is flexible and elastic and, like most metals, nitinol wires deform when subjected to minimal force and return to their shape in the absence of that force. Nitinol belongs to a class of materials called Shaped Memory Alloys (SMA) that have interesting mechanical properties beyond flexibility and elasticity, including shape memory and superelasticity which allow nitinol to have a "memorized shape" that is dependent on its temperature phases. The austenite phase is nitinol's stronger, higher-temperature phase, with a simple cubic crystalline structure. Superelastic behavior occurs in this phase (over a 50°-60°C temperature spread). Correspondingly, the martensite phase is a relatively weaker, lower-temperature phase with a twinned crystalline structure. When a nitinol material is in the martensite phase, it is relatively easily deformed and will remain deformed. However, when heated above its austenite transition temperature, the nitinol material will return to its pre-deformed shape, producing the "shape memory" effect. The temperature at which nitinol starts to transform to austenite upon heating is referred to as the "As" temperature. The temperature at which nitinol has finished transforming to austenite upon heating is referred to as the "Af" temperature. Accordingly, basket-shaped electrode assembly 16 when formed from such materials may have a three dimensional shape that can be easily collapsed to be fed into a guiding sheath and then readily returned to its expanded shape memory configuration upon delivery to the desired region of the patient upon removal of the guiding sheath. In one exemplary embodiment, the framework may be formed from a nitinol hypotube by laser cutting or other similar techniques, to provide a monolithic framework. For example, a 3mm tube having a wall thickness of approximately 8 to 9 mil may be used. Alternative embodiments may be formed from individual wires or other members.

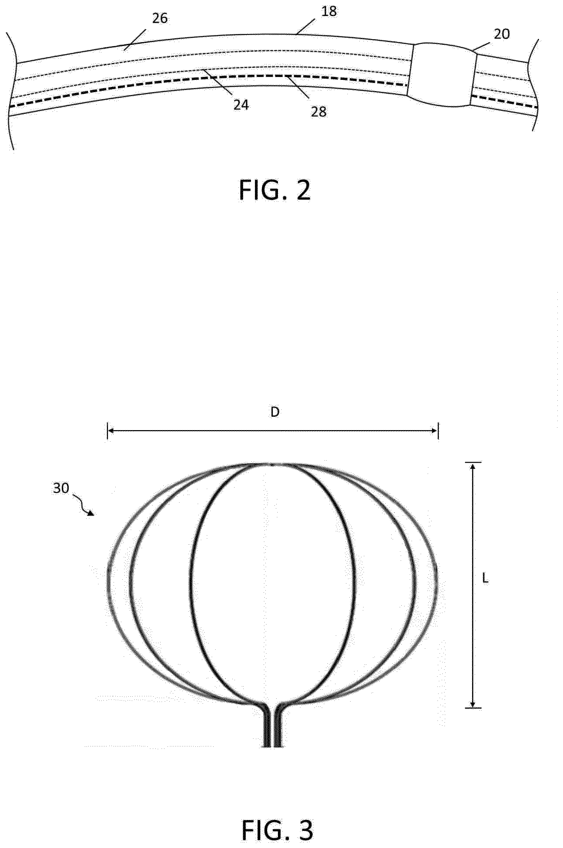

One example of a suitable construction is shown in FIG. 2, in which a portion of spine 18 is detailed. The framework of basket-shaped electrode assembly 16 forms a flexible core 24 that is surrounded by a non-conductive covering 26 on which one or more of the ring electrodes 20 are mounted. The non-conductive covering 26 may comprise a biocompatible plastic tubing, such as polyurethane or polyimide tubing, although other configurations may be employed. Further, cabling 28 may include lead wires for the electrodes 20 that are embedded or otherwise incorporated into non-conductive covering 26. In some embodiments, the techniques described in U.S. Application Serial No. 13/860,921, filed April 11, 2013, entitled HIGH DENSITY ELECTRODE STRUCTURE, and U.S. Application Serial No. 14/063,477, filed October 25, 2013, entitled CONNECTION OF ELECTRODES TO WIRES COILED ON A CORE, the entire disclosures of which are hereby incorporated by reference, may be employed. Cabling 28 may also include leads or conductors as necessary for other components that may be carried by spines 18, including temperature sensors, location sensors. In other embodiments, spines 18 may include other components, such as irrigation lumens, fiber optics, or others.

As noted, the framework of basket-shaped electrode assembly 16 may be preshaped so that is assumes an expanded arrangement upon deployment. However, the components carried by spines 18, such as the nonconductive covering 26, electrodes 20 and cabling 28 described above may resist the assumption of the intended preshaped configuration. For example, nonconductive covering 26 may tend to cause spines 18 to remain in the collapsed arrangement in which the spines are generally aligned with the longitudinal axis of catheter 10. In general, any components carried by spines 18 may hinder the assumption of the intended configuration when in the expanded arrangement. As an illustration, the framework may be preshaped with a spherical configuration, but due to the resistance created by the components carried by spines 18 (other than flexible core 24), spines 18 may not bow radially outwards to the amount necessary to achieve the spherical configuration, resulting in a more ellipsoidal expanded arrangement having a length greater than the diameter.

Accordingly, the techniques of this disclosure are directed to the use of a prestrained framework having a configuration that is tailored to overcome the resistance of the components of spines 18. In one embodiment, as shown in FIG. 3, a framework 30 for basket-shaped electrode assembly 16 that is intended to assume a spherical expanded arrangement may be prestrained to have a diameter D greater than length L. Other intended geometries of the expanded arrangement may be achieved by making the appropriate adjustments to the dimensions of the prestrained framework. In general, prestrained framework 30 may have a diameter D greater than the diameter and may have a length L less than the length of the intended expanded arrangement. The relative amount that the prestrained framework deviates from the intended expanded arrangement may be adjusted as warranted and may depend at least in part on the characteristics of the components carried by spines 18. Returning to the context of a basket-shaped electrode assembly 16 having an intended spherical expanded arrangement, framework 30 may have a D to L ratio in the range of approximately 2:1 to 6:5. For example, to achieve a spherical basket-shaped electrode assembly having a diameter of 48.2 mm, framework 28 may have a D of 60.9 mm and a L of 38.6 mm.

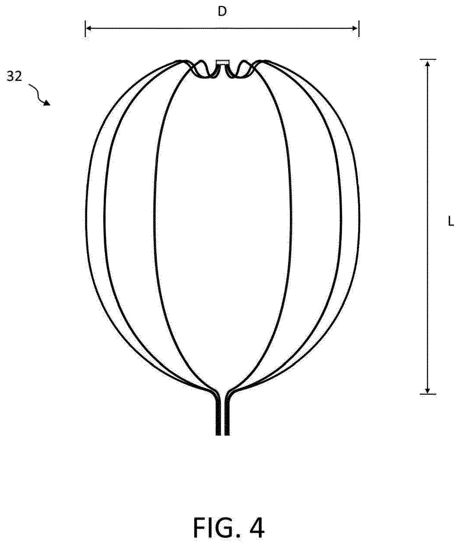

Another embodiment is shown in FIG. 4, in which framework 32 is prestrained with a length L equal to or greater than a diameter D, to provide a basket-shaped electrode assembly with an elliptical configuration. Incorporation of framework 32 into a basket-shaped electrode assembly again results in expanded arrangement that has a length greater than the prestrained length and a diameter less than the prestrained diameter due to the resistance imparted by the components on spines 18. For example, framework 32 may have a D to L ratio in the range of approximately 1:1 to 8:10. As will be appreciated, selecting an appropriate D:L ratio for the prestrained framework may overcome the resistance of the components on the spines to produce a basket-shaped electrode assembly having an expanded arrangement with a desired configuration. As an illustration, suitable D:L ratios for the prestrained framework for spherical and elliptical configurations may be in the range of approximately 2:1 to 8:10 and may result in basket-shaped electrode assemblies with expanded arrangements having D:L ratios in the range of approximately 3:2 to 7:10.

As will be appreciated, by employing a prestrained framework, the resulting basket-shaped electrode assembly will assume an expanded arrangement that more closely conforms to the intended configuration. In turn, the basket-shaped electrode assembly may deploy an electrode array that more completely covers the area in which it is positioned. Further, these benefits are obtained while retaining the same degree of flexibility. In comparison, attempts to overcome the resistance of the components carried by the spines by making the framework stiffer, such as by increasing the width and/or thickness of the flexible core, flexibility would be sacrificed, which may increase the risk of causing trauma to tissue that comes in contact with the basket-shaped electrode assembly as well as increasing the difficulty of constraining the spines into the collapsed arrangement for delivery and/or reducing the ability of the basket-shaped electrode assembly to navigate the tortuous anatomy of the patient's vasculature.

In one aspect, an electrophysiologist may introduce a guiding sheath, guidewire and dilator into the patient, as is generally known in the art. Examples of suitable guiding sheaths for use in connection with the inventive catheter are the PREFACE^{™} Braided Guiding Sheath (commercially available from Biosense Webster, Inc., Diamond Bar, CA) and the DiRex^{™} Guiding Sheath (commercially available from BARD, Murray Hill, NJ). The guidewire is inserted, the dilator is removed, and the catheter is introduced through the guiding sheath whereby the guidewire lumen in the pulling member permits the catheter to pass over the guidewire. In one exemplary procedure as depicted in FIG. 4, the catheter is first introduced to the right atrium (RA) via the inferior vena cava (IVC), where it passes through the septum (S) in order to reach the left atrium (LA).

As will be appreciated, guiding sheath 40 covers the spines 18 of the basket-shaped electrode assembly 16 in a collapsed position so that the entire catheter can be passed through the patient's vasculature to the desired location. Once the distal end of the catheter reaches the desired location, e.g., the left atrium, the guiding sheath is withdrawn to expose the basket-shaped electrode assembly 16. When unconstrained, spines 18 of basket-shaped electrode assembly 16 bow radially outwards to assume the expanded arrangement. By employing prestrained framework 30, the expanded arrangement more closely conforms to the intended configuration so that electrodes 20 are deployed into an array having more complete coverage. In particular, with the basket-shaped electrode assembly 16 radially expanded, electrodes 20 contact atrial tissue, allowing the electrophysiologist to map local activation time and/or ablate using electrodes 20.

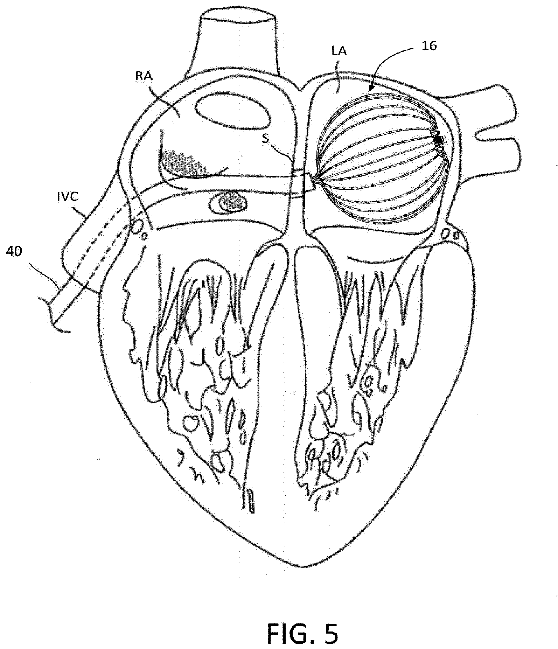

To help illustrate use of the basket-shaped electrode assembly 16, FIG. 5 is a schematic depiction of an invasive medical procedure, according to an embodiment of the present invention. Catheter 10, with the basket-shaped electrode assembly 16 (not shown in this view) at the distal end may have a connector 50 at the proximal end for coupling the wires from their respective electrodes 20 (not shown in this view) to a console 52 for recording and analyzing the signals they detect and/or for delivering energy to ablate tissue. An electrophysiologist 54 may insert the catheter 10 into a patient 56 in order to acquire electropotential signals from the heart 58 of the patient. The professional uses the control handle 14 attached to the catheter in order to perform the insertion. Console 52 may include a processing unit 60 which analyzes the received signals, and which may present results of the analysis on a display 62 attached to the console. The results are typically in the form of a map, numerical displays, and/or graphs derived from the signals.

In a further aspect, the processing unit 60 may also receive signals from one or more location sensors provided near a distal end of the catheter 10 adjacent the basket-shaped electrode assembly 16, such as location sensor 22. The sensor(s) may each comprise a magnetic-field-responsive coil or a plurality of such coils. Using a plurality of coils enables six-dimensional position and orientation coordinates to be determined. The sensors may therefore generate electrical position signals in response to the magnetic fields from external coils, thereby enabling processor 60 to determine the position, (e.g., the location and orientation) of the distal end of catheter 10 within the heart cavity. The electrophysiologist may then view the position of the basket-shaped electrode assembly 16 on an image the patient's heart on the display 62. By way of example, this method of position sensing may be implemented using the CARTO^{™} system, produced by Biosense Webster Inc. (Diamond Bar, Calif.) and is described in detail in U.S. Pat. Nos. 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference. As will be appreciated, other location sensing techniques may also be employed. If desired, at least two location sensors may be positioned proximally and distally with respect to electrode array assembly 16. The coordinates of the distal sensor relative to the proximal sensor may be determined and, with other known information pertaining to the configuration of basket-shaped electrode assembly 16, used to find the positions of each of the electrodes 20.

The preceding description has been presented with reference to presently disclosed embodiments of the invention. Workers skilled in the art and technology to which this invention pertains will appreciate that alterations and changes in the described structure may be practiced without meaningfully departing from the principal, spirit and scope of this invention. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

### Aspects of the invention

1. A method for treatment comprising:
   providing a catheter having an elongated catheter body with proximal and distal ends and a basket-shaped electrode assembly at the distal end of the catheter body, the basket-shaped electrode assembly comprising a plurality of spines connected at their proximal and distal ends, each spine comprising a plurality of electrodes, wherein the spines are formed by a framework which is prestrained to have a diameter and a length;
   advancing the distal end of the catheter with the basket-shaped electrode assembly to a desired region within a patient with the interconnected framework in a collapsed arrangement in which the spines are arranged generally along a longitudinal axis of the catheter body; and
   causing the basket-shaped electrode assembly to assume an expanded arrangement in which the spines bow radially outwards from the longitudinal axis of the catheter body so that at least one electrode is in contact with tissue, wherein the expanded arrangement has a length greater than the length of the prestrained framework and the expanded arrangement has a diameter less than the diameter of the prestrained framework.
2. The method of aspect 1, further comprising receiving electrical signals from the at least one electrode in contact with tissue.
3. The method of aspect 1, further comprising delivering radio frequency energy to the at least one electrode in contact with tissue to form a lesion.

## Claims

1. A catheter comprising an elongated catheter body having proximal and distal ends and a basket-shaped electrode assembly at the distal end of the catheter body, the basket-shaped electrode assembly comprising a plurality of spines connected at their proximal and distal ends, each spine comprising a plurality of electrodes, wherein the basket-shaped electrode assembly has an expanded arrangement having a length and a diameter in which the spines bow radially outward and a collapsed arrangement in which the spines are arranged generally along a longitudinal axis of the catheter body and wherein the spines are formed by a framework which is prestrained to have a diameter greater than the diameter of the expanded arrangement of the basket-shaped electrode assembly and a length less than the length of the expanded arrangement of the basket-shaped electrode assembly.

2. The catheter of claim 1, wherein the framework comprises a shape memory material.

3. The catheter of claim 2, wherein the framework is monolithic and formed from a cut tube of material.

4. The catheter of claim 1, wherein the spines further comprise a nonconductive covering.

5. The catheter of claim 4, wherein the length of the expanded arrangement is greater than the length of the prestrained framework and the diameter of the expanded arrangement is less than the diameter of the prestrained framework at least in part due to the nonconductive covering.

6. The catheter of claim 1, wherein the expanded arrangement of the basket-shaped electrode assembly has an approximately spherical configuration.

7. The catheter of claim 6, wherein the diameter of the prestrained framework is less than the length of the prestrained framework.

8. The catheter of claim 1, wherein the expanded arrangement of the basket-shaped electrode assembly has an approximately elliptical configuration.

9. A framework for a basket-shaped electrode assembly, comprising a plurality of flexible cores for spines of the basket-shaped electrode assembly, wherein the framework has a prestrained diameter greater than a diameter of an expanded arrangement of the basket-shaped electrode assembly and a prestrained length less than a length of an expanded arrangement of the basket-shaped electrode assembly.

10. The framework of claim 9, wherein the prestrained diameter of the framework is less than the prestrained length of the framework.

11. The catheter of claim 1 or the framework of claim 9, wherein the ratio of the diameter of the prestrained framework to the length of the prestrained framework is in the range of approximately 2:1 to 8:10.

12. A method for manufacturing a basket-shaped electrode with a length and a diameter when in an expanded arrangement and having a plurality of spines connected at their proximal and distal ends, comprising prestraining a framework to have a diameter greater than the diameter of the expanded arrangement of the basket-shaped electrode assembly and a length less than the length of the expanded arrangement of the basket-shaped electrode assembly, wherein the framework forms the spines of the basket-shaped electrode assembly.

13. The method of claim 12, further comprising applying components to the spines which cause the length of the expanded arrangement to be greater than the length of the prestrained framework and cause the diameter of the expanded arrangement to be less than the diameter of the prestrained framework.

14. The method of claim 13, wherein the components comprise a nonconductive covering for the spines.

15. The method of claim 12, wherein prestraining the framework comprises prestraining the framework with the diameter of the framework greater than the length of the framework so that the basket-shaped electrode assembly has an approximately spherical configuration when assuming the expanded arrangement.
